# FASCICULE DE BREVET EUROPEEN

(11) **EP 1 706 076 B1**
(45) Date de publication et mention de la délivrance du brevet: **13.06.2012**
(21) Numéro de dépôt: 05708206.7
(22) Date de dépôt: 07.01.2005
(51) Int. Cl.: A61F 2/44

(54) **PROTHESE DISCALE INTERVERTEBRALE**
BANDSCHEIBENPROTHESE
INTERVERTEBRAL DISCAL PROSTHESIS

(30) Priorité: 07.01.2004 FR 0400087; 10.06.2004 US 865487
(43) Date de publication de la demande: 04.10.2006
(73) Titulaire: SCIENT'X, 78284 Guyancourt (FR)
(72) Inventeur: CARLI, Olivier, CH-1207 Geneve (CH); BEN-MOKHTAR, Mourad, F-75018 Paris (FR)
(74) Mandataire: Thibault, Jean-Marc
(86) Numéro de dépôt international: PCT/FR2005/000034
(87) Numéro de publication internationale: WO 2005/067823

(56) Documents cités:
- EP-A- 0 955 021
- EP-A- 1 250 898
- WO-A-01/15638
- WO-A-03/059212
- US-A- 5 895 428
- US-B1- 6 517 580

## Description

La présente invention concerne d'une manière générale des implants vertébraux destinés à être utilisés dans le remplacement de disques intervertébraux, et plus particulièrement une prothèse discale articulée pour remplacer artificiellement le disque fibro-cartilagineux assurant la liaison entre les vertèbres de la colonne vertébrale.

Un disque intervertébral comprenant un élément déformable appelé « nucleus pulposus » entouré de plusieurs couches fibreuses élastiques, peut subir des altérations telles que compression, déformation, déplacement ou usure et plus généralement une dégénérescence associée à des contraintes mécaniques qui lui sont appliquées. Il peut en résulter une destruction anatomique et fonctionnelle du disque et du segment vertébral. Cette altération modifie le comportement mécanique du disque et aboutit à une diminution de la hauteur de l'espace intersomatique, laquelle entraîne une perturbation de l'ensemble de la fonction articulaire. Il en résulte une instabilité induisant, en particulier, une réaction arthrosique, source de douleurs et de processus ostéophytiques.

Il est bien connu de remplacer un disque déficient par un disque artificiel conçu pour tenter de reproduire la cinématique d'un mouvement naturel. Ainsi, le brevet US 5 562 738 décrit une prothèse discale comportant une première et une deuxième plaques réalisées en un matériau métallique tel qu'en titane pour la fixation aux vertèbres voisines. Entre les plaques est interposée une rotule d'articulation comportant un premier insert monté sur l'une des plaques et une calotte sphérique coopérant avec une cupule sphérique d'un deuxième insert monté sur l'autre plaque. Les inserts sont réalisés en un matériau céramique biocompatible présentant des caractéristiques tribologiques améliorées notamment en ce qui concerne sa résistance à l'usure.

Toutefois, une telle prothèse discale ne permet pas aux vertèbres de retrouver leur mobilité naturelle. En effet, une telle prothèse possède des débattements limités selon certains plans qui dépendent des formes dissymétriques de réalisation de la rotule d'articulation. La rotule d'articulation présente une forme relativement difficile à mener à bien et est sensible à la rupture ou à la fêlure, réduisant ainsi la durée de vie de la prothèse.

EP-A- 955 021 décrit une prothèse discale reproduisant les caractéristiques du préambule de la revendication 1.

Afin de réaliser une prothèse pour reproduire la cinématique du mouvement naturel, le brevet FR 2 730 159 décrit une prothèse comportant un noyau intermédiaire présentant deux faces sphériques orientées dans le même sens mais présentant des rayons différents. Un tel noyau intermédiaire est destiné à glisser sur une surface convexe appartenant à une plaque inférieure. Le noyau présente également une surface supérieure convexe sur laquelle glisse la plaque supérieure. Le noyau présente ainsi une mobilité dans le plan horizontal, de sorte qu'il est à même de s'écarter d'un côté lorsque les plaques se rapprochent de l'autre côté. Une telle prothèse présente toutefois l'inconvénient d'une éjection du noyau vers l'extérieur de la prothèse.

Pour interdire l'éjection du noyau de la prothèse, le brevet FR 2 659 226 décrit une prothèse dont la plaque supérieure présente une face concave qui vient glisser sur un noyau polyéthylène en forme de calotte sphérique, immobilisé dans une cavité de la plaque inférieure. Les plaques inférieure et supérieure en titane sont munies chacune d'ailerons d'ancrage dans une vertèbre respective pour éviter tout risque de désassemblage. Des collerettes à double redans limitent le débattement angulaire des plaques inférieure et supérieure au moyen de sections complémentaires sur le pourtour de la plaque supérieure et sur le pourtour de la plaque inférieure. En pratique, les collerettes à double redans sont destinées à venir s'imbriquer l'une dans l'autre lors du décalage angulaire maximum entre les plaques, ce qui peut conduire à un emboîtement avec frottement indésirable, voire à un blocage. De plus, une telle prothèse impose une implantation dans les plaques vertébrales relativement traumatisante et ne permet pas d'assurer le transfert optimal de la poussée de la vertèbre supérieure sur la vertèbre inférieure. Enfin, l'interface de contact entre un noyau polyéthylène et une plaque en titane se dégrade dans le temps et modifie ainsi de manière indésirable la mobilité de la prothèse.

La présente invention vise à remédier à de tels inconvénients en proposant une prothèse de disque intervertébral pour vertèbres qui reproduit fidèlement les mouvements naturels du disque et assure une transmission optimale de la poussée de la vertèbre supérieure sur la vertèbre inférieure tout en offrant un contrôle du débattement angulaire relatif entre les vertèbres.

Un autre objet de l'invention est de proposer une prothèse de disque vertébral rétablissant la fonction physiologique normale dans la colonne vertébrale en préservant le mouvement intervertébral, la stabilité, la lordose, et l'espacement tout en protégeant les structures vasculaires, nerveuses et les autres structures vertébrales.

Un mode de réalisation de la présente invention propose une prothèse qui peut comporter une première et une deuxième plaques destinées à être fixées à des vertèbres voisines, et une rotule d'articulation interposée entre les deux plaques montées en position superposée, de manière que les faces internes de ces plaques se trouvent tournées l'une vers l'autre. La rotule peut comprendre une calotte sphérique coopérant avec une cupule sphérique. La première plaque, dite plaque porteuse ici, possède dans le plan frontal, une dimension supérieure à la dimension correspondante de la deuxième plaque, de manière à constituer une butée pour la deuxième plaque, et par sa face externe une surface de portée pour la vertèbre plus importante que celle présentée par la deuxième plaque.

La prothèse discale peut avoir en outre dans le plan horizontal une forme sensiblement trapézoïdale dont la grande base délimite le bord antérieur de la plaque tandis que la petite base délimite le bord postérieur. La deuxième plaque peut avoir dans le plan horizontal une forme parallélépipédique.

Les plaques peuvent être dimensionnées de telle manière que :
E/d est au moins approximativement égal à E/D₁ pour A₁/A₂ > 0,5, avec E : distance entre les deux faces internes des plaques ; d : largeur de la plaque supérieure dans le plan frontal ; D₁ : longueur de la plaque supérieure dans le plan sagittal ; A₁ : angle maximal de débattement entre les plaques dans le plan frontal ; et A₂ : angle maximal de débattement entre les plaques dans le plan sagittal.

Par exemple, au moins l'un des angles parmi A₁ et A₂ ne dépasse pas 10 degrés.

La première plaque peut avoir une face interne de profil plan dans le plan frontal et une face externe de profil convexe dans le plan frontal. L'une des plaques peut inclure, sur sa face interne, un anneau élastique de contact avec la face interne de l'autre plaque.

Pour une compréhension plus totale de l'invention, on se réfère à la description détaillée qui suit, prise en combinaison avec les dessins annexés illustrant différents modes de réalisation de la présente invention, dans lesquels :
La **Figure 1** est une vue de face dans le plan frontal d'un premier exemple de réalisation d'une prothèse selon l'invention en position médiane.
La **Figure 2** est une vue en coupe dans le plan frontal de la prothèse selon l'invention, en position inclinée.
La **Figure 3** est une vue de côté dans le plan sagittal d'une prothèse selon l'invention en position médiane.
La **Figure 4** est une vue en coupe dans le plan sagittal d'une prothèse selon l'invention en position inclinée.
La **Figure 5** est une vue de dessus dans le plan horizontal ou coronal d'une prothèse selon l'invention.
Les **Figures 6** et **7** sont des vues en coupe respectivement dans le plan frontal et dans le plan sagittal d'un deuxième exemple de réalisation d'une prothèse selon l'invention en position inclinée.
Les **Figures 8** et **9** sont des vues en coupe respectivement dans le plan frontal et dans le plan sagittal d'un troisième exemple de réalisation d'une prothèse selon l'invention en position inclinée.
Les **Figures 10** et **11** sont des vues de face et de côté d'un autre mode de réalisation de la prothèse discale de la **Fig. 1****.**
La **Figure 12** est une vue en coupe transversale prise selon la ligne 12-12 de la **Fig. 10****.**
La **Figure 12A** est une vue partiellement éclatée d'une partie absorbant les chocs illustrée à la **Fig. 12****.**
Les **Figures 13** et **14** sont des vues de face et de côté d'un autre mode de réalisation de la prothèse discale de la **Fig. 10****.**
La **Figure 15** est une vue en coupe transversale prise suivant la ligne 15-15 de la **Fig. 13****.**

La présente invention va maintenant être décrite plus complètement en se référant aux dessins annexés dans lesquels d'autres modes de réalisation de l'invention sont montrés et décrits. Il convient de comprendre que l'invention peut être concrétisée sous de nombreuses formes différentes et ne devrait pas être considérée comme limitée aux modes de réalisation illustrés présentés ici. Au contraire, ces modes de réalisation sont fournis de manière que cette divulgation puisse être totale et complète, et feront apparaître le cadre de l'invention à l'homme du métier.

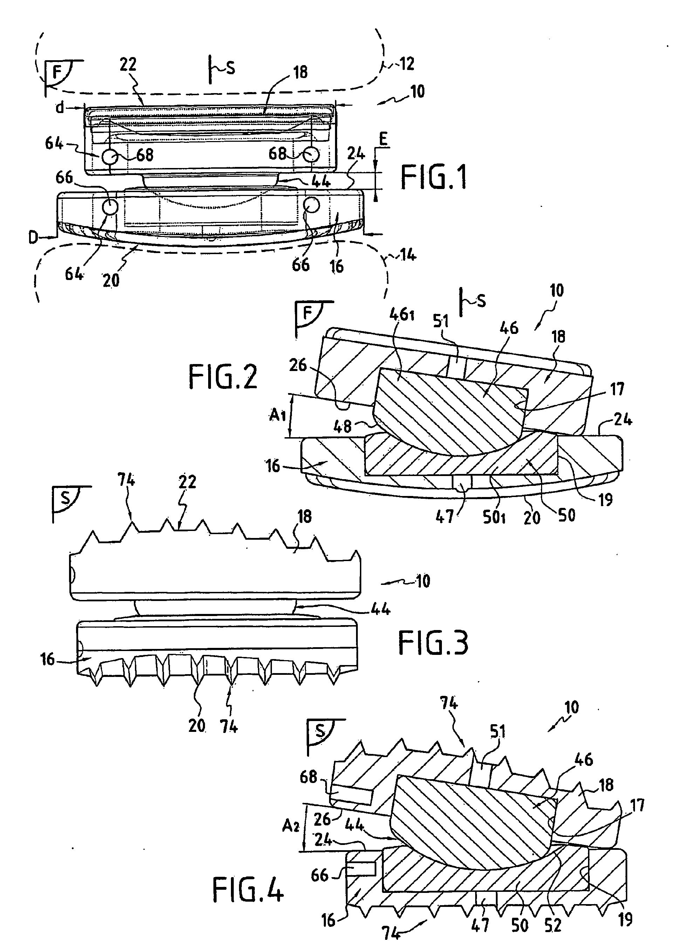

En référence initialement aux **Fig. 1 - 4****,** un mode de réalisation de la présente invention peut être décrit comme une prothèse discale **10** destinée à être implantée à la place d'un disque intervertébral entre deux vertèbres adjacentes **12, 14.** Un mode de réalisation de la prothèse discale **10** décrite ici, à titre d'exemple, inclut une première plaque **16** représentée comme étant une plaque inférieure dans l'exemple illustré et une deuxième plaque **18** représentée comme étant une plaque supérieure. Les plaques **16, 18** sont destinées à être fixées aux vertèbres adjacentes **12, 14** et sont décrites ici, à titre d'exemple, comme ayant chacune une face externe, **20, 22,** respectivement, et ayant chacune une face interne, **24, 26,** respectivement, s'étendant en vis-à-vis l'une de l'autre. A titre d'exemple, les plaques **16, 18** peuvent être réalisées en titane ou en alliage de titane. Des modes de réalisation des première et deuxième plaques **16, 18,** telles que décrites ici à titre d'exemple, peuvent être formés en un matériau biométallique, et avoir éventuellement un revêtement ostéoconducteur. Dans un mode de réalisation, les première et deuxième plaques comprennent du titane avec un revêtement d'hydroxyapatite.

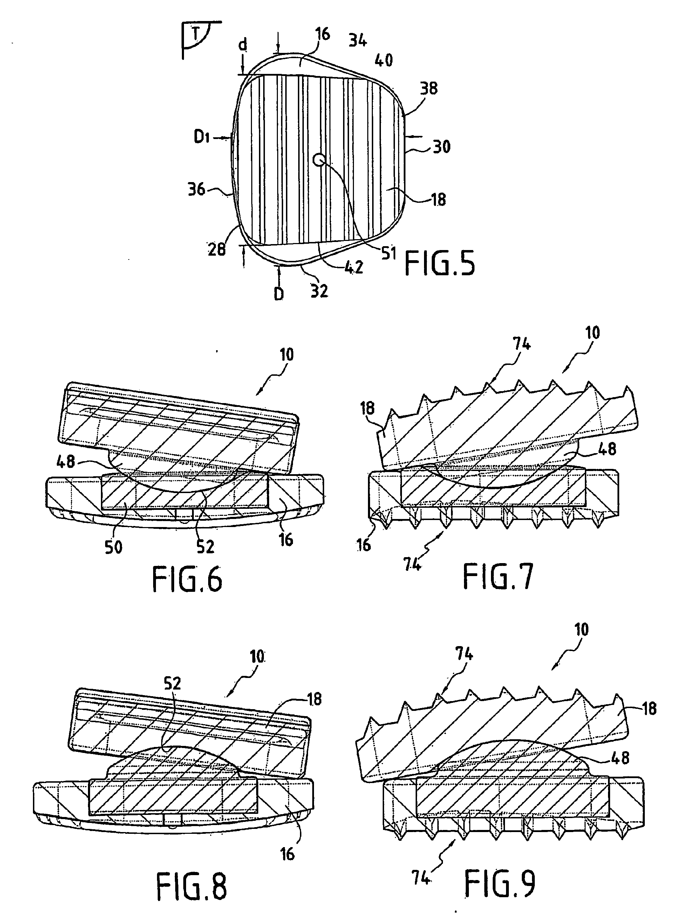

En continuant à se référer à la **Fig. 1** et à la **Fig. 5****,** la première plaque **16,** connue également comme étant une plaque porteuse, possède une dimension transversale **D** supérieure à la dimension **d** correspondante de la deuxième plaque **18.** Ces dimensions **d, D** sont mesurées dans une direction transversale à l'intersection entre le plan horizontal **T,** illustré en se référant de nouveau à la **Fig. 5****,** et le plan frontal **F,** illustré en se référant de nouveau à la **Fig. 1****,** et sont pris en considération ici en termes de plans anatomiques bien connus.

En se référant aux **Fig. 2** et **4****,** la face interne **24** de la première plaque **16** constitue ainsi une butée pour la deuxième plaque **18** et avec la face externe **20** de la première plaque **16** une surface de portée pour la vertèbre **14** qui est plus importante que la surface de la vertèbre **12** avec laquelle entre en contact la surface externe **22** présentée par la deuxième plaque **18.** Comme cela est illustré en se référant de nouveau à la **Fig. 5**, la première plaque **16,** la plaque porteuse, possède dans le plan horizontal **T** une forme sensiblement trapézoïdale dont la grande base délimite un bord antérieur **28** tandis que la petite base délimite un bord postérieur **30.** Le bord postérieur **30** est raccordé au bord antérieur **28** par des bords opposés divergents **32, 34.** Des congés de raccordement peuvent relier de préférence les bords antérieur et postérieur **28, 30** aux bords divergents **32, 34.**

Comme cela est illustré en se référant de nouveau aux **Fig.1** et **2** pour un mode de réalisation décrit ici à titre d'exemple, la première plaque **16** possède une face interne **24** de profil plan et une face externe **20** de profil convexe dans le plan frontal **F.** En se référant de nouveau à la Fig. **5****,** la deuxième plaque **18** possède dans le plan horizontal **T** une forme sensiblement parallélépipédique avec un bord antérieur **36** et un bord postérieur **38** qui sont sensiblement parallèles et confondus en position de superposition avec le bord antérieur **28** et le bord postérieur **30** de la première plaque **16.** Le bord antérieur **36** de la deuxième plaque **18** est raccordé au bord postérieur **38** par l'intermédiaire de deux bords de liaison **40, 42** sensiblement parallèles entre eux. Des congés de raccordement peuvent relier les bords antérieur et postérieur **36, 38** aux bords de liaison **40, 42.** Tel que cela ressort clairement en se référant de nouveau aux **Fig. 1, 2** et **5****,** la première plaque **16** déborde ainsi dans le plan horizontal **T** de part et d'autre des bords de liaison **40, 42** de la deuxième plaque **18.**

En continuant à se référer aux **Fig. 1 - 4****,** la prothèse discale **10** décrite ici comporte une rotule d'articulation **44** interposée entre les deux plaques **16, 18** qui sont superposées l'une sur l'autre. Dans l'exemple de réalisation illustré sur les **Fig.** 1 à **5****,** la rotule d'articulation **44** comprend des surfaces incurvées coopérantes incluant un premier insert **46** présentant une calotte sphérique **48** et un deuxième insert **50** présentant une cupule d'un logement sphérique **52** coopérant en douceur avec la calotte sphérique **48.** Chaque insert **46, 50** est monté dans un logement **17, 19,** par exemple un logement borgne, au travers de la face interne **26, 24** de chaque plaque **18, 16.** Chaque insert **46, 50** possède une forme générale de révolution et présente respectivement une base **46₁, 50₁** de section droite transversale circulaire dont l'une des extrémités est aménagée pour présenter la calotte sphérique **48** ou la cupule sphérique **52.** Dans un mode de réalisation, les inserts **46, 50** sont réalisés en un matériau céramique ou en polyéthylène.

Comme cela est illustré en se référant de nouveau aux **Fig. 2** et **4****,** à titre d'exemple, des trous d'évent **47, 51** sont prévus dans les première et deuxième plaques **16, 18,** respectivement, et s'étendent depuis les logements **19, 17** formées dans les première et deuxième plaques **16, 18.** Les trous d'évent **47, 51** permettent à l'air de s'échapper en les traversant lors de l'insertion des inserts **46, 50** dans les logements **17, 19** pendant un procédé de fabrication.

Il est clair d'après la description qui précède que la première plaque **16,** la plaque porteuse, est plus large que la deuxième plaque **18** dans la direction transversale de manière à constituer d'une part une surface plane de butée, la surface interne **24,** pour la deuxième plaque **18** et d'autre part une grande surface de portée, la face externe **20,** avec la vertèbre associée. Comme cela ressort clairement en outre en se référant de nouveau aux **Fig. 2** et **4****,** les positions de butée de la prothèse, aussi bien dans le plan frontal **F** que dans le plan sagittal **S**, sont déterminées par la mise en contact des plaques **16,18** l'une avec l'autre.

Comme cela est illustré en outre en se référant de nouveau aux **Fig. 1 - 5****,** un mode de réalisation de la prothèse discale **10** peut être dimensionné de telle manière que : E/d est approximativement égal à E/D₁ pour A₁/A₂ > 0,5, où E représente la distance entre les deux faces internes **24, 26** des plaques **16, 18 ;** d représente la largeur de la deuxième plaque (supérieure) **18** dans le plan frontal **F ;** D₁ représente la longueur de la deuxième plaque (supérieure) **18** dans le plan sagittal **S ;** A₁ représente l'angle maximal de débattement entre les plaques **16, 18** dans le plan frontal **F ;** et **A₂** représente l'angle maximal de débattement entre les plaques **16, 18** dans le plan sagittal **S.** Pour l'exemple de la prothèse discale **10,** décrite ici à titre d'exemple, les angles A sont d'environ dix degrés ou moins mais peuvent être plus grands si on le souhaite pour une utilisation particulière sans s'écarter de l'enseignement de la présente invention. Un tel dimensionnement permet de fournir une prothèse discale à débattements contrôlés qui correspondent sensiblement aux mouvements naturels du disque vertébral.

Dans l'exemple illustré en se référant aux **Fig. 1** à **5****,** la calotte sphérique **48** et la cupule sphérique **52** sont réalisées sur des inserts **46, 50** fixés aux plaques **18, 16.** A titre d'alternative, et comme cela est illustré en se référant aux **Fig. 6** et **7****,** un mode de réalisation constituant une autre variante dans laquelle la calotte sphérique **48** fait partie intégrante de la deuxième plaque **18,** la plaque supérieure dans l'exemple illustré. Pour ce mode de réalisation, la calotte sphérique **48** peut être en titane tandis que la cupule sphérique **52** est aménagée dans un insert **50** réalisé en céramique ou en polyéthylène.

De plus, dans les exemples qui précèdent, la calotte sphérique **48** est montée sur la deuxième plaque (supérieure) **18** tandis que la cupule sphérique **52** est portée par la première plaque (inférieure) **16.** A titre d'alternative, et en se référant aux **Fig. 8** et **9****,** un mode de réalisation représentant une autre variante peut inclure la cupule sphérique **52** portée par la deuxième plaque (supérieure) **18** tandis que la calotte sphérique **48** est portée par la première plaque (inférieure) **16.** En outre également, et à titre d'exemple, un mode de réalisation peut inclure des surfaces d'articulation zircone sur alumine pour la calotte sphérique **48** et la cupule sphérique **52** de la rotule d'articulation **44.** A titre d'alternative, le contact surface sur surface de la calotte et de la cupule peut être zircone sur zircone ou alumine sur alumine, à titre d'exemple.

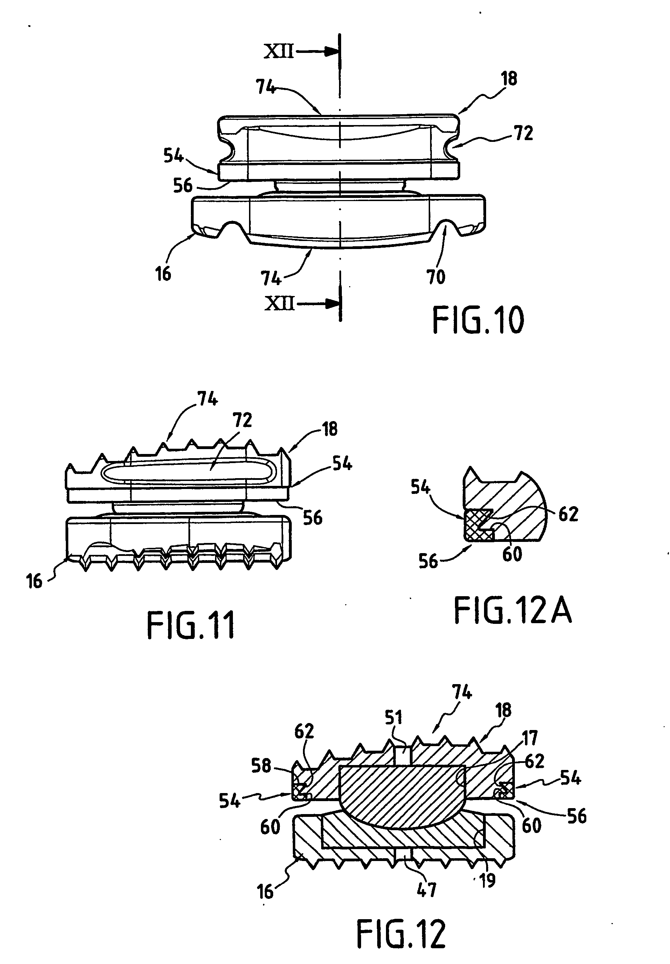

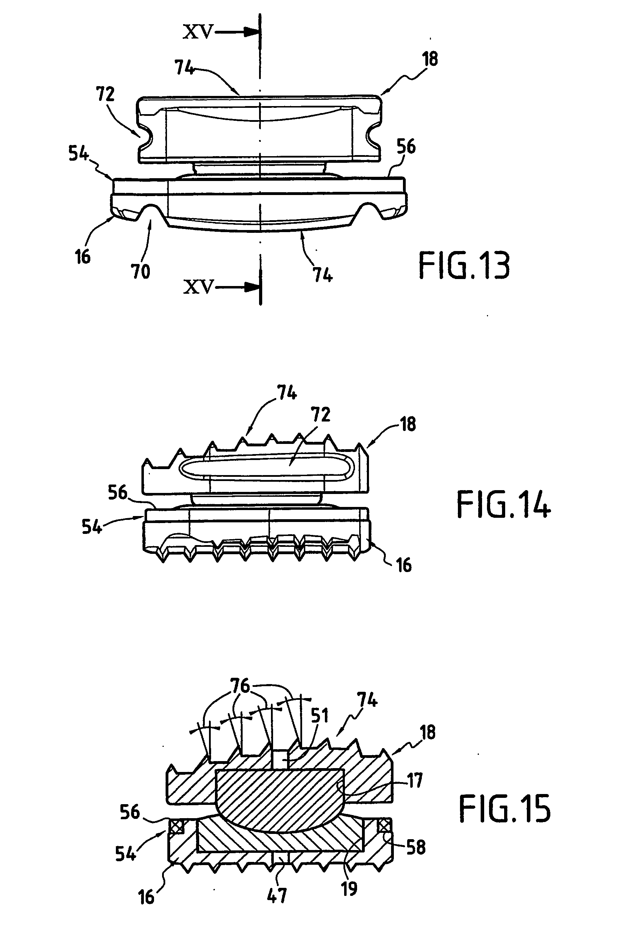

En outre, et en se référant aux **Fig. 10 - 15****,** à titre d'exemple, la première plaque **16** ou la deuxième plaque **18** peut porter un matériau absorbant les chocs **54** destiné à entrer en contact avec la plaque opposée **16, 18** pour les plaques d'articulation en butée de mouvement. Ainsi que cela est représenté ici à titre d'exemple, le matériau absorbant peut être un anneau élastique **56** qui s'étend autour de et qui est porté en contact de frottement dans les parties périphériques des faces internes **24, 26** des plaques **16, 18.** Ainsi que cela est illustré ici à titre d'exemple, l'anneau élastique **56** peut être monté dans une gorge **58** entourant la calotte sphérique **48** et/ou la cupule sphérique **52** et disposée sur la face interne **24, 26.** Dans un mode de réalisation, les plaques **16, 18** peuvent inclure une encoche ou gorge **58** qui s'étend sur le périmètre pour recevoir l'anneau élastique **56** à l'intérieur. En outre, et comme cela est illustré en se référant à la **Fig. 12****,** l'anneau élastique **56** peut inclure une rainure interne **60** pour recevoir une patte **62** dans une paroi de la gorge **58** pour retenir l'anneau élastique **56** dans la gorge **58.**

Ainsi que cela est illustré en se référant de nouveau aux **Fig. 1** et **4****,** un mode de réalisation de la prothèse discale **10** peut comprendre des moyens de positionnement **64,** comme les trous **66** dans la surface antérieure de la première plaque **16** et les trous **68** portés dans la deuxième plaque **18** pour recevoir un outil pour maintenir simultanément les plaques. A titre d'alternative, et comme cela est illustré en se référant aux **Fig. 10, 11****,** **13** et **14****,** les moyens de positionnement **64** peuvent comprendre une paire de rainures parallèles **70, 72** portées par chacune des première et deuxième plaques **16, 18** respectivement, pour recevoir l'outil. A titre d'exemple supplémentaire, et en continuant à se référer aux **Fig. 10 - 15****,** les paires de rainures parallèles **70, 72** pour les première et deuxième plaques **16, 18** peuvent s'étendre de leurs parties antérieures à leurs parties postérieures, la première plaque incluant les rainures **70** le long de la face externe **20** et la deuxième plaque **18** incluant les rainures **72** le long de ses bords de liaison.

Pour les modes de réalisation de la prothèse discale **10** décrits ci-dessus, les faces externes **20, 22** pour au moins l'une des première et deuxième plaques **16, 18** comprennent une pluralité de dents **74** pour maintenir les plaques entre les vertèbres adjacentes **12, 14.** Ainsi que cela est illustré à titre d'exemple en se référant de nouveau à la **Fig. 5****,** au moins une partie de la pluralité de dents peut comprendre un déplacement de pente antérieur pour faciliter une insertion dans un espace entre des vertèbres tout en limitant le mouvement antérieur des plaques **16, 18.**

De nombreuses modifications et d'autres modes de réalisation de l'invention viendront à l'esprit de l'homme du métier ayant l'avantage des enseignements présentés dans la description précédente et les dessins annexés. De ce fait, il convient de comprendre que l'invention ne doit pas être limitée aux modes de réalisation spécifiques décrits, et que des modifications et d'autres modes de réalisation sont destinés à être inclus dans le cadre des revendications annexées.

## Revendications

1. Prothèse discale **(10)** comprenant :
- une première plaque **(16)** pour la fixation à une première vertèbre,
- une deuxième plaque **(18)** pour la fixation à une deuxième vertèbre adjacente, et
- une rotule d'articulation **(4)** interposée entre les première et deuxième plaques **(16, 18)** montées l'une au dessus de l'autre, de sorte que les faces internes **(24, 26)** des plaques **(16, 18)** sont tournées l'une vers l'autre, la rotule d'articulation **(44)** comportant une calotte sphérique **(48)** coopérant avec une partie comprenant une cupule sphérique **(52),**
- une dimension transversale **(D)** de la première plaque **(16)** étant supérieure à une dimension **(d)** correspondante de la deuxième plaque **(18),** de manière à constituer, par la face interne **(24)** de la première plaque **(16),** une butée pour la deuxième plaque **(18)** et, par la face externe **(20)** de la première plaque **(16),** une surface de portée pour les vertèbres qui est plus grande qu'une surface de portée de la face externe **(22)** de la deuxième plaque **(18)**
**caractérisé en ce que** les première et deuxième plaques **(16, 18)** sont dimensionnées de telle manière que :
E/d est approximativement égal à E/D₁ pour A₁/A₂ > 0,5.
avec
E : distance entre les deux faces internes **(24, 26)** des plaques **(16, 18),**
d : largeur de la plaque supérieure **(18)** dans le plan frontal **(F),**
D₁ : longueur de la plaque supérieure **(18)** dans le plan sagittal,
A₁ : angle maximal de débattement entre les plaques **(16, 18)** dans le plan frontal, et
A₂ : angle maximal de débattement entre les plaques **(16, 18)** dans le plan sagittal.

2. Prothèse discale selon la revendication 1, **caractérisée en ce que** la première plaque **(16)** comprend une forme sensiblement trapézoïdale dans un plan horizontal **(T),** où une grande base de la première plaque **(16)** définit un bord antérieur **(28)** et où une petite base de celle-ci définit un bord postérieur **(30).**

3. Prothèse discale selon la revendication 1 ou 2, **caractérisée en ce que** la deuxième plaque **(18)** est sensiblement en forme de parallélépipède dans le plan horizontal **(T).**

4. Prothèse discale selon la revendication 1, **caractérisée en ce qu'**au moins l'un des angles parmi A₁ et A₂ ne dépasse pas dix degrés.

5. Prothèse discale selon la revendication 1, **caractérisée en ce que** la première plaque **(16)** possède une face interne **(24)** de profil plan dans un plan frontal **(F)** et une face externe **(20)** de profil convexe dans le plan frontal **(F).**

6. Prothèse discale selon la revendication 1, **caractérisée en ce que** les première et deuxième plaques **(16, 18)** comprennent un matériau biométallique ayant un revêtement ostéoconducteur.

7. Prothèse discale selon la revendication 1, **caractérisée en ce que** les surfaces d'articulation de la calotte sphérique **(48)** et de la cupule sphérique **(52)** pour la rotule d'articulation **(44)** comprennent au moins un matériau parmi la zircone sur alumine, la zircone sur zircone et l'alumine sur alumine.

8. Prothèse discale selon la revendication 1, **caractérisée en ce que** la calotte sphérique **(48)** comprend un matériau de titane et la cupule sphérique **(52)** comprend au moins un matériau parmi un matériau céramique et un matériau polyéthylène.

9. Prothèse discale selon la revendication 1, **caractérisée en ce que** l'une au moins parmi les première et deuxième plaques **(16, 18)** porte un matériau absorbant les chocs **(54)** sur la face interne **(24, 26)** pour entrer en contact avec une plaque opposée.

10. Prothèse discale selon la revendication 9, **caractérisée en ce que** le matériau absorbant les chocs **(54)** comprend un anneau élastique **(56)** qui s'étend sur un périmètre d'au moins une plaque parmi les première et deuxième plaques **(16, 18).**

11. Prothèse discale selon la revendication 9, **caractérisée en ce qu'**elle comprend une gorge **(58)** dans au moins une plaque parmi les première et deuxième plaques **(16, 18)** pour porter à l'intérieur le matériau absorbant les chocs **(54).**

12. Prothèse discale selon la revendication 1, **caractérisée en ce qu'**elle comprend au moins des moyens de positionnement **(64)** portés dans chacune des première et deuxième plaques **(16, 18)** pour recevoir un outil pour maintenir les plaques **(16, 18)** simultanément.

13. Prothèse discale selon la revendication 12, **caractérisée en ce que** le ou les moyens de positionnement **(64)** comprennent un trou **(66, 68)** porté dans des parties antérieures pour chacune des première et deuxième plaques **(16, 18).**

14. Prothèse discale selon la revendication 12, **caractérisée en ce que** le ou les moyens de positionnement **(64)** pour chacune des première et deuxième plaques **(16, 18)** comprennent une paire de rainures parallèles **(70, 72)** portées par chacune des première et deuxième plaques **(16, 18)** pour recevoir un outil pour maintenir les plaques **(16, 18)** simultanément.

15. Prothèse discale selon la revendication 14, **caractérisée en ce que** la paire de rainures parallèles **(70, 72)** pour les première et deuxième plaques **(16, 18)** s'étend de leurs parties antérieures à leurs parties postérieures, et où la première plaque **(16)** inclut les rainures **(70)** le long de la face externe **(20)** et la deuxième plaque **(18)** inclut les rainures **(72)** le long de ses bords de liaison.

16. Prothèse discale selon la revendication 1, **caractérisé en ce que** la face externe **(20, 22)** pour au moins l'une des première et deuxième plaques **(16, 18)** comprend une pluralité de dents **(74)** pour maintenir les plaques **(16, 18)** entre des vertèbres adjacentes.

17. Prothèse discale **(10)** selon la revendication 16, **caractérisée en ce qu'**au moins une partie de la pluralité de dents **(74)** comprend un déplacement de pente antérieur **(76)** pour faciliter une insertion dans un espace entre des vertèbres tout en limitant le mouvement antérieur des plaques **(16, 18).**

## Claims

1. A disk prosthesis comprising:
- a first plate (16) for attaching to a first vertebrae;
- a second plate (18) for attaching to a second adjacent vertebrae; and
- a ball and socket joint (4) interposed between the first and second plates (16, 18) stacked one on top of the other, so that inner faces (24, 26) of the plates (16, 18) are turned toward one another, wherein a ball portion of the ball and socket joint (44) comprises a spherical cap (48) cooperating with a socket portion of the joint (44) comprising a spherical cup (52),
- a transversal dimension (D) of the first plate (16) is greater than a corresponding dimension (d) of the second plate (18), so as to constitute, by the inner face (24) of the first plate (16), a limit stop for the second plate (18) and, by the outer face (20) of the first plate (16), a bearing surface for the vertebrae that is larger than a bearing surface of the outer face (22) of the second plate (18),
**characterized in that** the first and second plates (16, 18) are dimensioned such that:
E/d at least approximately equal to E/D₁ for A₁/A₂ > 0.5,
with E: distance between the two inner faces (24, 26) of plates (16,18),
d: width of the upper plate (18) in the frontal plane (F),
D₁: length of the upper plate (18) in the sagittal plane,
A₁: maximum angle of clearance between plates (16, 18) in the frontal plane, and
A₂: maximum angle of clearance between plates (16, 18) in the sagittal plane.

2. A disk prosthesis according to Claim 1, **characterized in that** the first plate (16) comprises a substantially trapezoidal shape in a horizontal plane (T), wherein a large base of the first plate (16) defines a leading edge (28), and wherein a small base thereof defines a trailing edge (30).

3. A disk prosthesis according to Claims 1 or 2, **characterized in that** the second plate (18) is substantially shaped as a parallelepiped in the horizontal plane (T).

4. A disk prosthesis according to Claim 1, wherein at least one of angles among A1 and A2 does not exceed ten degrees.

5. A disk prosthesis according to Claim 1, **characterized in that** the first plate (16) has an inner face (24) with a flat profile in a frontal plane (F) and an outer face (20) with a convex profile in the frontal plane (F).

6. A distal prosthesis according to Claim 1, wherein the first and second plates (16,18) comprise a biometallic material having an osteoconductive coating.

7. A distal prosthesis according to Claim 1, wherein articulating surfaces of the spherical cap (48) and the spherical cup (52) for the ball and socket joint (44) comprise at least one of a zirconia-on-alumina, zirconia-on-zirconia, and alumina-on-alumina material.

8. A disk prosthesis according to Claim 1, wherein the spherical cap (48) comprises a titanium material and the spherical cup (52) comprises at least one of a ceramic material and a polyethylene material.

9. A disk prosthesis according to Claim 1, **characterized in that** at least one of the first and second plates (16, 18) carries a shock absorbing medium (54) on the inner face (24, 26) for making contact with an opposing plate.

10. A disk prosthesis according to Claim 9, wherein the shock absorbing medium (54) comprises an elastic ring (56) extending about a perimeter of at least one of the first and second plates (16, 18).

11. A disk prosthesis according to Claim 9, further comprising a recess (58) within at least one of the first and second plates (16, 18) for carrying the shock absorbing medium (54) therein.

12. A disk prosthesis according to Claim 1, further comprising at least one positioning cutout (64) carried within each of the first and second plates (16, 18) for receiving a tool for simultaneously holding the plates (16,18).

13. A disk prosthesis according to Claim 12, wherein the at least one positioning cutout (64) comprises a hole (66, 68) carried within anterior portions for each of the first and second plates (16,18).

14. A disk prosthesis according to Claim 12, **characterized in that** the at least one positioning cutout (64) for each of the first and second plates (16, 18) comprises a pair of parallel grooves (70, 72) carried by each of the first and second plates (16, 18) for receiving a tool for simultaneously holding the plates (16, 18).

15. A disk prosthesis according to Claim 14, **characterized in that** the pair of parallel grooves (70, 72) for the first and second plates (16, 18) extend from anterior to posterior portions thereof, and wherein the first plate (16) includes the grooves (70) along the outer face (20) and the second plate (18) includes the grooves (72) along opposing side wall surfaces thereof.

16. A disk prosthesis according to Claim 1, **characterized in that** the outer face (20, 22) for at least one of the first and second plates (16, 18) comprises a plurality of teeth (74) for retaining the plates (16, 18) between the adjacent vertebrae.

17. A distal prosthesis according to Claim 16, **characterized in that** at least a portion of the plurality of teeth (74) comprises an anterior slope bias (76) for facilitating an insertion into a space between vertebrae while restricting anterior distraction of the plates (16, 18).

## Patentansprüche

1. Bandscheibenprothese (10), umfassend:
- eine erste Platte (16) für die Befestigung an einem ersten Wirbel,
- eine zweite Platte (18) für die Befestigung an einem benachbarten zweiten Wirbel und
- einen Gelenkkugelkopf (44), der zwischen der ersten und der zweiten Platte (16, 18), welche übereinander angebracht sind, eingefügt ist, so daß die Innenflächen (24, 26) der Platten (16, 18) einander zugewandt sind, wobei der Gelenkkugelkopf (44) eine Kugelkappe (48) umfaßt, die mit einem eine Kugelschale (52) umfassenden Teil zusammenwirkt,
- wobei eine Querabmessung (D) der ersten Platte (16) größer als eine entsprechende Abmessung (d) der zweiten Platte (18) ist, um durch die Innenfläche (24) der ersten Platte (16) einen Anschlag für die zweite Platte (18) und durch die Außenfläche (20) der ersten Platte (16) eine Auflagefläche für die Wirbel zu bilden, die größer als eine Auflagefläche der Außenfläche (22) der zweiten Platte (18) ist,
**dadurch gekennzeichnet, daß** die erste und die zweite Platte (16, 18) derart dimensioniert sind, daß:
E/d annähernd gleich E/D₁ bei A₁/A₂ > 0,5 ist,
mit E: Abstand zwischen den beiden Innenflächen (24, 26) der Platten (16, 18),
d: Breite der oberen Platte (18) in der Frontalebene (F),
D₁: Länge der oberen Platte (18) in der Sagittalebene,
A₁: maximaler Ausstellungswinkel zwischen den Platten (16, 18) in der Frontalebene und
A₂: maximaler Ausstellungswinkel zwischen den Platten (16, 18) in der Sagittalebene.

2. Bandscheibenprothese nach Anspruch 1, **dadurch gekennzeichnet, daß** die erste Platte (16) in einer Horizontalebene (T) im wesentlichen eine Trapezform aufweist, wobei eine große Basis der ersten Platte (16) eine vordere Kante (28) und eine kleine Basis derer eine hintere Kante (30) definiert.

3. Bandscheibenprothese nach Anspruch 1 oder 2, **dadurch gekennzeichnet, daß** die zweite Platte (18) in der Horizontalebene (T) im wesentlichen quaderförmig ausgebildet ist.

4. Bandscheibenprothese nach Anspruch 1, **dadurch gekennzeichnet, daß** wenigstens einer der Winkel aus A₁ und A₂ zehn Grad nicht übersteigt.

5. Bandscheibenprothese nach Anspruch 1, **dadurch gekennzeichnet, daß** die erste Platte (16) eine Innenfläche (24) mit einem in einer Frontalebene (F) ebenen Profil sowie eine Außenfläche (20) mit einem in der Frontalebene (F) konvexem Profil besitzt.

6. Bandscheibenprothese nach Anspruch 1, **dadurch gekennzeichnet, daß** die erste und die zweite Platte (16, 18) einen Biometall-Werkstoff mit einer osteokonduktiven Beschichtung umfassen.

7. Bandscheibenprothese nach Anspruch 1, **dadurch gekennzeichnet, daß** die Gelenkflächen der Kugelkappe (48) und der Kugelschale (52) für den Gelenkkugelkopf (44) wenigstens ein Material aus Zirkonoxid auf Aluminiumoxid, Zirkonoxid auf Zirkonoxid und Aluminiumoxid auf Aluminiumoxid umfassen.

8. Bandscheibenprothese nach Anspruch 1, **dadurch gekennzeichnet, daß** die Kugelkappe (48) ein Titanmaterial und die Kugelschale (52) wenigstens ein Material aus einem Keramikmaterial und einem Polyethylenmaterial umfaßt.

9. Bandscheibenprothese nach Anspruch 1, **dadurch gekennzeichnet, daß** wenigstens eine der ersten und zweiten Platten (16, 18) ein stoßdämpfendes Material (54) auf der Innenfläche (24, 26) trägt, um mit einer gegenüberliegenden Platte in Kontakt zu gelangen.

10. Bandscheibenprothese nach Anspruch 9, **dadurch gekennzeichnet, daß** das stoßdämpfende Material (54) einen elastischen Ring (56) umfaßt, der sich über einen Umfang von wenigstens einer Platte der ersten und zweiten Platten (16, 18) erstreckt.

11. Bandscheibenprothese nach Anspruch 9, **dadurch gekennzeichnet, daß** sie eine Vertiefung (58) in wenigstens einer Platte der ersten und zweiten Platten (16, 18) umfaßt, um innen das stoßdämpfende Material (54) zu tragen.

12. Bandscheibenprothese nach Anspruch 1, **dadurch gekennzeichnet, daß** sie wenigstens Positionierungsmittel (64), die in jeder der ersten und zweiten Platten (16, 18) angeordnet sind, umfaßt, um ein Werkzeug aufzunehmen, um die Platten (16, 18) gleichzeitig zu halten.

13. Bandscheibenprothese nach Anspruch 12, **dadurch gekennzeichnet, daß** das oder die Positionierungsmittel (64) ein in vorderen Teilen ausgebildetes Loch (66, 68) für jede der ersten und zweiten Platten (16, 18) umfassen.

14. Bandscheibenprothese nach Anspruch 12, **dadurch gekennzeichnet, daß** das oder die Positionierungsmittel (64) für jede der ersten und zweiten Platten (16, 18) ein Paar von parallelen Nuten (70, 72), die an jeder der ersten und zweiten Platten (16, 18) ausgebildet sind, umfassen, um ein Werkzeug aufzunehmen, um die Platten (16, 18) gleichzeitig zu halten.

15. Bandscheibenprothese nach Anspruch 14, **dadurch gekennzeichnet, daß** das Paar von parallelen Nuten (70, 72) für die erste und die zweite Platte (16, 18) sich von ihren vorderen Teilen zu ihren hinteren Teilen erstreckt, und wobei die erste Platte (16) die Nuten (70) entlang der Außenfläche (20) und die zweite Platte (18) die Nuten (72) entlang ihrer Verbindungskanten einschließt.

16. Bandscheibenprothese nach Anspruch 1, **dadurch gekennzeichnet, daß** die Außenfläche (20, 22) bei wenigstens einer der ersten und zweiten Platten (16, 18) eine Vielzahl von Zähnen (74) umfaßt, um die Platten (16, 18) zwischen benachbarten Wirbeln zu halten.

17. Bandscheibenprothese (10) nach Anspruch 16, **dadurch gekennzeichnet, daß** wenigstens ein Teil der Vielzahl von Zähnen (74) eine vordere Neigungsversetzung (76) aufweist, um ein Einfügen in einen Raum zwischen Wirbeln zu erleichtern und gleichzeitig die vordere Bewegung der Platten (16, 18) zu begrenzen.
